# EUROPEAN PATENT SPECIFICATION

(11) **EP 2 048 092 B1**
(45) Date of publication and mention of the grant of the patent: **27.02.2013**
(21) Application number: 07744899.1
(22) Date of filing: 07.06.2007
(51) Int. Cl.: A61F 13/14, A61F 13/551

(54) **PACKED BREAST MILK ABSORBENT PAD AND METHOD FOR PACKING**
VERPACKTE ABSORBIERENDE STILLEINLAGE UND VERFAHREN ZUR VERPACKUNG
COUSSINET DE POITRINE ABSORBANT EMBALLÉ ET PROCÉDÉ D'EMBALLAGE

(30) Priority: 08.06.2006 JP 2006160350
(43) Date of publication of application: 15.04.2009
(73) Proprietor: UNI-CHARM CORPORATION, Shikokuchuo-shi Ehime 799-0111 (JP)
(72) Inventor: KAWAKAMI, Hikari, Kanonji-shi, Kagawa 7691602 (JP); FUJIKAWA, Michiyo, Kanonji-shi, Kagawa 7691602 (JP)
(74) Representative: Stark, Gordon Drummond
(86) International application number: PCT/JP2007/061571
(87) International publication number: WO 2007/142307

(56) References cited:
- WO-A1-88/10219
- GB-A- 2 221 667
- JP-A- 07 313 547
- JP-A- 10 127 688
- JP-A- 2000 309 391
- JP-A- 2004 059 065
- US-A- 5 792 131
- US-A1- 2002 183 709
- US-A1- 2003 014 032
- US-A1- 2004 077 473
- US-A1- 2005 015 067

## Description

### TECHNICAL FIELD

The present invention relates a packed breast milk absorbent pad and a method for packing.

### RELATED ART

There have conventionally been proposed packed breast milk absorbent pads provided in the form of an assembly comprising a breast milk absorbent pad itself and an individual pad packing envelope wherein the pad itself is composed of an absorbent structure having its outer surface covered with a leak-barrier sheet and the pad packing envelope temporarily fastens the pad packed therein.

For example, JP2000-309391A, JP3657537B1 and JP2001-88866A mentioned later disclose the packed breast milk absorbent pad comprising the individual packing envelope and the twofold breast milk absorbent pad packed therein, the packed breast milk absorbent pad comprising the elongated band-like individual packing envelope and a pair of breast milk absorbent pads packed therein side-by-side in the same orientation and the packed breast milk absorbent pad comprising the individual packing envelope and a pair of breast milk absorbent pads packed therein so as to be stacked upon each other in opposite orientations.
GB 2221667, US 2002/183709, US 5792131, WO 88/10219 and US 2003/014032 disclose panty liners wrapped in individual wrappings. US 2004/077473 discloses an interlabial pad wrapped in an individual wrapping. US 2005/015067 describes an individually wrapped absorbent body.

### DISCLOSURE OF THE INVENTION

### PROBLEMS TO BE SOLVED BY THE INVENTION

However, in any of the packed breast milk absorbent pads disclosed in JP 2000-309391A, JP 3657537B1 and JP 2001-88866A, neither the end nor the side edge of the breast milk absorbent pad packed in the individual packing envelope is present in the vicinity of the pad unpacking outlet. Consequentially, the heat-sealed edge of the individual packing envelope must be broken to pick out the pad from the individual packing envelope.

The individual packing envelope having been broken is no more useful, after the breast milk absorbent pad has been used, to put again the used pad thereinto for disposal.

An object of the present invention is to provide a packed breast milk absorbent pad and a method of packing allowing the breast milk absorbent pad to be easily unpacked from the individual packing envelope so that the same individual packing envelope can be used as the envelope to put again the used pad thereinto for disposal.

### MEASURE TO SOLVE THE PROBLEMS

The present invention to achieve the object set forth above has two aspects.

The present invention provides, in an aspect thereof, an improvement in a packed breast milk absorbent pad comprising a breast milk absorbent pad having upper and lower ends, a skin-contactable surface as well as a skin-noncontactable surface and an individual packing envelope packing the breast milk absorbent pad therein.

The first aspect of the present invention is defined by features as will be described below.

The individual packing envelope is formed of a heat-sealable packing sheet having a longitudinal direction, a transverse direction, first and second side edges extending in the longitudinal direction, and first and second ends extending in the transverse direction wherein the packing sheet is divided by first and second folds extending in the transverse direction from the first edge to the second edge and spaced apart from each other in the longitudinal direction into first, second and third regions.

The breast milk absorbent pad has first and second sections defined by folding the pad into two with the skin-contactable surface inside, and a temporary fastening zone formed by adhesive on the skin-noncontactable surface of the first section aside toward the upper end in order to enable fastening of the breast milk absorbent pad to a brassiere.

The first region of the packing sheet is folded back toward the skin-noncontactable surface in the first section of the breast milk absorbent pad, and fastened, in this folded state, to the temporary fastening zone by a peel ply provided on an inner surface of the first region of the packing sheet opposed to the temporary fastening zone, and the third region of the packing sheet is folded back toward the second region, so that a distal end of said third region extends between said first region and said second region of said breast milk absorbent pad.

The first, second and third regions are sealed together along the first and second side edges of the packing sheet to form seal lines.

A second aspect of the present invention to solve the object set forth above provides a method for packing a breast milk absorbent pad comprising a breast milk absorbent pad having upper and lower ends, a skin-contactable surface as well as a skin-noncontactable surface into an individual packing envelope formed of a packing sheet.

The second aspect of the present invention comprises steps as will be described below.

Providing, as stock material for the individual packing envelope, a heat-sealable packing sheet having a longitudinal direction, a transverse direction, first and second side edges extending in the longitudinal direction, and first and second ends extending in the transverse direction.

Defining first, second and third regions on the packing sheet by first and second folds extending in the transverse direction from the first edge to the second edge and spaced apart from each other in the longitudinal direction.

Laying the breast milk absorbent pad upon the packing sheet so as to extend astride the first fold in the longitudinal direction and thereby to lie on both the first and second regions of the packing sheet, then folding the third region of the packing sheet along the second fold toward the second region, and thereafter folding the first region of the packing sheet together with the breast milk absorbent pad along the first fold toward the second region so as to fold the breast milk absorbent pad into first and second sections with said skin-contactable surface inside, whereupon the first region of the packing sheet is temporarily fixed to the skin-noncontactable surface of the breast milk absorbent pad in the first section thereof aside toward the upper end by intermediary of a temporary fastening zone formed by a pressure-sensitive adhesive on the skin-noncontactable surface to enable temporary fixing of the breast milk absorbent pad to a brassiere, and by intermediary of a peel ply formed on the first region of the packing sheet on the surface thereof opposed to the temporary fastening zone.

Sealing the first and second regions of the packing sheet together along the first and second side edges thereof so as to form seal lines.

The first aspect of the present invention includes preferred embodiments as will be described below.

The third region of the packing sheet is folded back toward the second region of the sheet so that a distal end of the third region extends between the first section and the second section of the breast milk absorbent pad.

The peel ply is formed from a release coated sheet permanently fastened to the inner surface of the first region of the packing sheet by means of a permanent fastening zone formed by adhesive.

The peel ply is formed by coating of adhesive.

An end of the permanent fastening zone for the releasable sheet aside toward the upper end of the breast milk absorbent pad extends outward beyond an end of the temporary fastening zone aside toward the upper end of the breast milk absorbent pad.

A peel strength of the releasable sheet with respect to the temporary fastening zone is in a range of 0.1 to 0.5 N and a peel strength of the seal along the first and second side edges of the packing sheet is in a range of 1.0 to 2.26, N.

The first end of the packing sheet defines an unpacking outlet of the breast milk absorbent pad, wherein the unpacking outlet is temporarily bonded to an outer surface of the third region of the sheet.

The first end of the packing sheet defines an unpacking outlet of the breast milk absorbent pad wherein the unpacking outlet is free rather than being temporarily bonded to the outer surface of the third region of said sheet.

According to the second aspect of the present invention, the seal is preferably provided in the form of heat seal.

### EFFECT OF THE INVENTION

The packed breast milk absorbent pad according to the present invention primarily **characterized in that** a temporary fastening zone of the breast milk absorbent pad with respect to the individual packing envelope, i.e., in a vicinity of the pad unpacking outlet in order that the breast milk absorbent pad alone can be easily taken out from the envelope merely by pulling the upper end of the breast milk absorbent pad. For further benefit, there is no or substantially no possibility that the envelope might be torn when the pad is unpacked from the envelope and, after the pad has been used, the envelope can be utilized as the envelope for the used pad for disposal.

In the embodiment wherein the peel ply is formed by coating of adhesive, the peel ply can be formed without use of the releasable sheet and the cost can be correspondingly saved.

In the embodiment wherein an end of the permanent fastening zone for the releasable sheet aside toward the upper end of the breast milk absorbent pad extends outward beyond an end of the temporary fastening zone aside toward the upper end of the breast milk absorbent pad, it is ensured that the breast milk absorbent pad can be easily peeled off from the envelope so as to leave the releasable sheet on the peel ply of the envelope when the breast milk absorbent pad is unpacked from the envelope.

In the embodiment wherein a peel strength of the releasable sheet with respect to the temporary fastening zone is in a range of 0.1 to 0.5 N and a peel strength of the seal along the first and second side edges of the packing sheet is in a range of 1.0 to 2.2 N, it is unlikely that the sealed zones might be unsealed or the envelope might be torn when the breast milk absorbent pad is detached from the releasable sheet and unpacked from the envelope.

The packing method according to the present invention conveniently permits the breast milk absorbent pad to be folded into two at the same time the first region of the packing envelope is folded onto the second region of the packing envelope. Compared to the case in which the breast milk absorbent is previously folded into two, the process of packing is advantageously simplified.

In the embodiment wherein the seal is provided in the form of heat seal, the cost can be lowered in comparison with the case in which the sealing is achieved by use of adhesive.

In the embodiment wherein the first end of the packing sheet defining the unpacking outlet for the breast milk absorbent pad is temporarily bonded to the outer surface of the third region of the sheet, there is no anxiety that dust might enter into the pad after the step of bonding in the process of making the pad.

In the embodiment wherein the first end of the packing sheet defining the unpacking outlet for the breast milk absorbent pad is free from the outer surface of the third region of the sheet, the step of bonding the first end is not required.
The invention provides a packed breast milk absorbent pad and a method for packing a breast milk absorbent pad as defined in the appended claims.

### BRIEF DESCRIPTION OF THE DRAWINGS

[Fig. 1] Fig. 1 is a partially cutaway perspective view of a packaged breast milk absorbent pad.
[Fig. 2] Fig. 2 is a sectional view taken along the line II-II in Fig. 1.
[Fig. 3] Fig. 3 is a plan view showing the breast milk absorbent paid with a skin-noncontactable surface thereof lying in the figure's plane.
[Fig. 4] Fig. 4 is a developed plan view of an envelope for individually packing the pad.
[Fig. 5] Fig. 5 is a schematic sectional view showing another embodiment of the packaged pad.
[Fig. 6] Fig. 6 is a schematic sectional view showing still another embodiment of the packaged pad.
[Fig. 7] Fig. 7 is a sectional view similar to Fig. 2, showing further another embodiment.

### IDENTIFICATION OF REFERENCE NUMERALS USED IN THE DRAWINGS

- 1: packaged breast milk absorbent pad
- 2: breast milk absorbent pad
- 2A: first section
- 2B: second section
- 3: packing envelope
- 3A: packing sheet
- 11: temporary fastening zone
- 11a: end
- 13: permanent fastening zone
- 13a: end
- 12: releasable sheet
- 15a: first side edge
- 15b: second side edge
- 16a: first end
- 16b: second end
- 17: first fold
- 18: second fold
- 19: first region
- 20: second region
- 21: third region
- 23: pressure-sensitive adhesive zone
- 24: pad unpacking outlet
- 26a, 26b: seal line
- 30: peel ply

### DESCRIPTION OF THE BEST MODE FOR WORKING OF THE INVENTION

The present invention will be described hereunder on the basis of preferred embodiments with reference to the accompanying drawings.

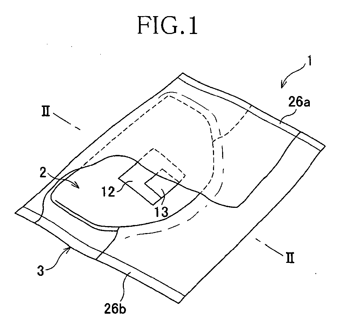

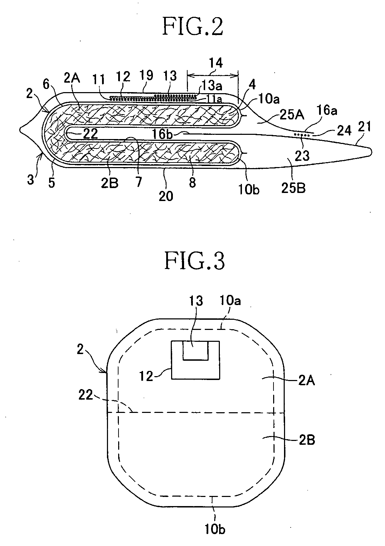

Fig. 1 is a partially cutaway perspective view of a packaged breast milk absorbent pad, Fig. 2 is a sectional view taken along the line II-II in Fig. 1 and Fig. 3 is a plan view showing the breast milk absorbent pad with a skin-noncontactable surface lying in the figure's plane.

Referring to Fig. 1, a packaged breast milk absorbent pad 1 comprises a breast milk absorbent pad 2 and an individual packing envelope 3.

Referring now to Fig. 2, the breast milk absorbent pad 2 comprises a body fluid absorbent layer 4 and a body fluid leak-barrier sheet 5 (i.e., outer sheet). The absorbent layer 4 comprises, in turn, a body fluid absorbent structure 6 and a body fluid pervious inner sheet 7 covering an inner surface of the body fluid absorbent structure 6. The absorbent structure 6 comprises, in turn, a body fluid absorbent core 8.

The core 8 comprises fluff pulp fibers as a base material and super-absorbent polymer particles or the like mixed with the fluff pulp fibers so as to form a panel having a basis weight preferably in a range of 200 to 300 g/m². While not illustrated, it is particularly preferred to cover the core 8 with a body fluid spreadable shape retaining sheet in order to prevent the polymer particles from falling off.

The inner sheet 7 is laid so as to face and come in contact with the wearer's skin and made of a thermoplastic synthetic fibrous nonwoven fabric, preferably such as a spun bond or air-through nonwoven fabric having a basis weight preferably in a range of 10 to 35 g/m². The inner sheet 7 is intermittently coated with a hot melt adhesive (not shown) by which the inner sheet 7 is kept to cover the absorbent structure 6.

The skin-noncontactable surface of the absorbent structure 6 is covered with the body fluid leak-barrier sheet (i.e., the outer sheet) 5 which is preferably made of a plastic film having a thickness in a range of 30 to 100 µm or a thermoplastic synthetic fibrous nonwoven fabric, particularly a spun bond/melt blown/spun bond (SMS) laminated nonwoven fabric having a basis weight of 10 to 35 g/m², or a composite sheet consisting of these plastic film and nonwoven fabric.

The breast milk absorbent pad 2 is folded into two in such a manner that upper and lower ends 10a, 10b (See Fig. 3) are placed upon each other and the inner sheet 7 lies inside so as to define first and second sections 2A, 2B. The outer sheet 5 in the first section 2A is provided aside toward the upper end 10a of the pad 2 with a temporary fastening zone 11 formed by a pressure-sensitive adhesive (hot melt adhesive) in order that the pad may be reliably held against any undesirable displacement with respect to a brassiere. The temporary fastening zone 11 is covered with a releasable sheet 12 which is, in turn, permanently fixed to the inner surface of the individual packing envelope 3 by a permanent fastening zone 13 formed by a hot melt adhesive. In this way, the temporary fastening zone 11 is separable from the releasable sheet 12. A finger grip zone 14 is defined between the upper end 10a and the temporary fastening zone 11. This finger grip zone 14 is appropriately dimensioned so that the upper end 10a of the breast milk absorbent pad 2 can be held by the wearer's fingers without touching the temporary fastening zone 11 when the wearer's unpacks the breast milk absorbent pad 2 from the envelope.

An end 13a of the permanent fastening zone 13 on the side of the upper end 10a of the pad 2 preferably extends beyond an end 11a of the permanent fastening zone 11 toward the upper end 10a of the pad 2. This is for the reason as follows: Intending to unpack the pad 2 out from the envelope 3 by gripping the upper end 10a with the fingers of the wearer's one hand, the wearer may often hold a first region 19 of the envelope 3 with the fingers of the other hand. In this case, a peel force is exerted directly upon the end 11a of the temporary fastening zone 11 in the course of unpacking the pad 2 from the envelope 3 since the end 13a of the permanent fastening zone 13 extends beyond the end 11a of the temporary fastening zone 11 toward the upper end 10a of the pad 2. In other words, assumed that the end 11a of the temporary fastening zone 11 extends beyond the end 13a of the permanent fastening zone 13 toward the upper end 10a of the pad 2, the peel force will be exerted upon the end 13a of the permanent fastening zone 13 lying adjacent to the upper end 10a of the pad 2 and make it difficult to peel the temporary fastening zone 11 off from the releasable sheet 12.

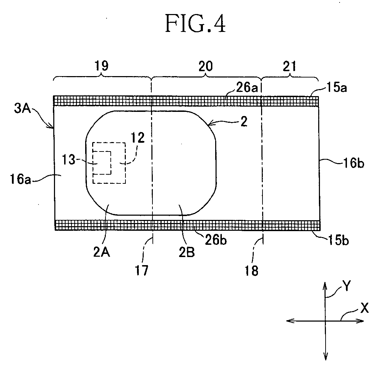

Fig. 4 is a developed plan view of the individual packing envelope 3. As will be apparent from Fig. 4, the individual packing envelope 3 is made of a flexible and heat-sealable packing sheet 3A which is substantially rectangular as viewed in a direction X. The individual packing envelope 3 is contoured by first and second side edges 15a, 15b, first and second ends 16a, 16b, and divided into imaginary first, second and third regions 19, 20, 21 divided by imaginary first and second folds 17, 18 spaced apart one from another in the direction X. The third region 21 has a dimension in the direction X smaller than the first and second regions 19, 20 have.

The packing sheet 3A preferably comprises a plastic film having a thickness in a range of 30 to 100 µm, or preferably comprises a thermoplastic synthetic fibrous nonwoven fabric having a basis weight in a range of 10 to 35 g/m², particularly such as a spun bond/melt blown/spun bond (SMS) laminated nonwoven fabric, or a laminate consisting of these plastic film and nonwoven fabric.

Using the individual packing envelope 3 formed from the packing sheet 3A in the manner as has been described above, the breast milk absorbent pad 2 is packed therein in a manner as will be described below.

Referring to Fig. 2 and Fig. 4, the breast milk absorbent pad 2 is placed upon the packing sheet 3A so that a fold 22 (See Fig. 3) along which the breast milk absorbent pad 2 is to be folded into two may lie just on the first fold 17 of the packing sheet 3A, the first and second sections 2A, 2B of the breast milk absorbent pad 2 may lie respectively on the first and second regions 19, 20 of the packing sheet 3A and the temporary fastening zone 11 may be fixed to the permanent fastening zone 13 previously formed on the packing sheet 3A by intermediary of the releasable sheet 12. From such a state, the third region 21 of the packing sheet 3A is folded back with a distal end of this third region 21 placed on the second section 2B of the breast milk absorbent pad 2, then the assembly is folded into two so that the first region 19 of the packing sheet 3A may be folded back toward the second region 20 and simultaneously the first section 2A of the pad 2 may be folded back just above the second section 2B of the pad 2.

The packing sheet 3A having been folded in this manner is then subjected to a heat-sealing treatment along the first and second side edges 15a, 15b to form seal lines 26a, 26b and the first end 16a of the packing envelope 3A is temporarily bonded to the opposed outer surface of the third region 21 of the packing sheet 3A by a pressure-sensitive adhesive zone 23 formed by hot melt adhesive so as to define a pad unpacking outlet 24.

In the embodiment according to which the first end 16a defining the unpacking outlet 24 is sealed by the pressure-sensitive adhesive zone 23, it is unlikely that dust might enter into the envelope.

Alternatively, the first end 16a may be free instead of bonding to the outer surface of the third region 21.

In this case, the step of bonding the first end 16a to the third region 21 may be saved.

The individual packing envelope 3 is formed from the packing sheet 3A in the manner as has been described above and then the pad 2 is packed therein in the twofold state. In the state of the pad 2 packed in this manner, first and second access sites 25A, 25B are defined within the envelope 3 so that respective portions of the pad 2 in vicinities of the upper and lower ends 10a, 10b thereof may occupy these access sites 25A, 25B.

The pad 2 packed in the envelope 3 in this manner may be unpacked from the bag 3, for example, by handling as will be described below (See Fig. 2). Specifically, the wearer may hold the first end 16a of the first region 19 and pull this first end 16a upward as viewed in Fig. 2 to peel the adhesive zone 23 off in the embodiment according to which the first region 19 and the third region 21 are bonded together along the ends thereof (handling of such peeling off is not required in the embodiment according to which the adhesive zone 23 is not provided) until an opening defined between the first and third regions 19, 21 are sufficiently broadened. Then the upper end 10a of the pad 2 may be held and pulled rightward as viewed in Fig. 2 to unpack the pad 2 from the envelope 3 through the pad unpacking outlet 24 (the first case).

Alternatively, without further broadening the opening defined between the first and third regions 19, 21, the wearer's fingers may be inserted into the envelope 3 through the unpacking outlet 24, the upper end 10a of the pad 2 may be held with the wearer's fingers and pulled rightward as viewed in Fig. 2 to unpack the pad 2 through the pad unpacking outlet 24 (the second case). In this case, a relatively high shearing force is exerted on the first region 19 instead of the peel force as in the first case. In view of this, it is preferred to use materials having a relative high tensile strength as the packing sheet 3A.

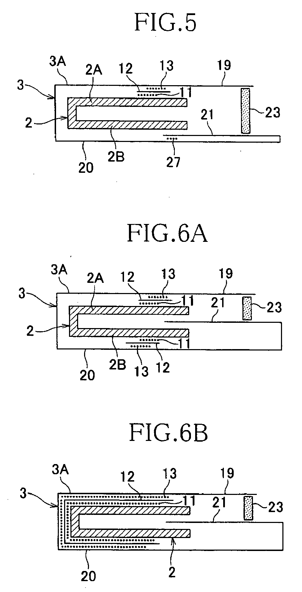

In an embodiment schematically illustrated by Fig. 5 is distinguished from the embodiment shown in Fig. 2 in that the third region 21 underlies the pad 2. In this case, it is possible to bond the third region 21 to the second region 20 by hot melt adhesive 27.

Fig. 6 is a schematic sectional view of the packed breast milk absorbent pad. Referring to Fig. 6A, the pad 2 is provided in the first and second sections 2A, 2B with the temporary fastening zones 11 while referring to Fig. 6B, the temporary fastening zone 11 continuously extends in the longitudinal direction.

The temporary fastening zone 11 may extend over the substantially entire outer surface of the pad 2 except the first end 16a of the packing sheet 3A so far as at least a part thereof is present in a vicinity of the upper end 10a of the pad 2. While not illustrated, a plurality of the temporary fastening zones 11 may be arranged along at least one of the upper and lower ends 10a, 10b of the pad 2 in the transverse direction of in the longitudinal direction so far as at least a part thereof is present in the vicinity of the upper end 10a of the pad 2.

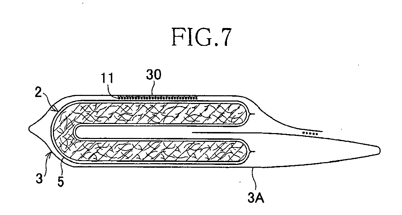

Fig. 7 is a sectional view similar to Fig. 2, illustrating another embodiment. The packing sheet 3A is coated on its inner surface with a release agent such as silicone to form a peel ply 30 and the temporary fastening zone 11 is fixed directly to this peel ply 30. In this case, the releasable sheet 12 is not necessary for actual use of the pad.

For bonding and attachment of the respective components, adhesives such as hot melt adhesive and pressure-sensitive adhesive, and heat-sealing techniques may selectively utilized.

A peel strength of the releasable sheet 12 with respect to the temporary fastening zone 11 is preferably in a range of 0. 1 to 0.5 N allowing the pad 2 to be peeled off from the envelope 3 without damaging the sealed line along the first and second side edges 15a, 15b of the packing sheet 3A.

A peel strength of the seal lines 26a, 26b along the first and second side edges 15a, 15b of the packing sheet 3A is preferably in a range of 1.0 to 2.2 N. If the peel strength is 1.0 N or less, there is a possibility that the seal lines 26a, 26b might be broken at the same time as seal of the pad unpacking outlet 24 is broken in order to unpack the pad 2. If the peel strength is 2.2 N or higher, there is an anxiety that the packing sheet 3A might be damaged.

The peel strength is measured using Universal Material Tester available from INSTRON JAPAN by a method as will be described below.

### <Peel Strength of the releasable sheet with respect to the temporary fastening zone>

After the product has been left for 30 min at a temperature of 20°C and a humidity of 60% for stabilization, the seal line 26 is removed from the product, the first region 19 is cut away. The upper end 10a of the pad 2 and the first end 16a of the packing sheet 3A fastened to the pad 2 or the end of the releasable sheet 12 aside toward the pad unpacking outlet 24 are held by chucks (a distance between chucks: 20 mm and a tensile rate: 500 mm/min) to measure the maximum load. Five measurements according to this method are repeated and an average value is obtained as the peel strength of the releasable sheet 12 with respect to the temporary fastening zone 11.

### <Peel Strength of the seal line>

After the product has been left for 30 min at a temperature of 20°C and a humidity of 60% for stabilization, a test piece defined by a dimension of 25 mm measured from the seal line 26 at the pad unpacking outlet 24 in a direction in which the seal line 26 is to be peeled off and a dimension of 75 mm measured from the seal line 26 toward a center of the product is cut away from the product. Of the test piece having cutaway from the product in this manner, the first and second ends 16a, 16b of the packing sheet 3A are held by chucks (a distance between chucks: 30 mm and a tensile rate: 300 mm/min) to measure the maximum load. Five measurements according to this method are repeated and an average value is obtained as the peel strength of the seal line 26.

## Claims

1. A packed breast milk absorbent pad (1) comprising:
a breast milk absorbent pad (2) having upper and lower ends (10a, 10b), a skin-contactable surface (7) as well as a skin-noncontactable surface (5); said pad being **characterized in that**:
an individual packing envelope (3) packing said breast milk absorbent pad therein is formed of a heat-sealable packing sheet (3A) having a longitudinal direction (X), a transverse direction (Y), first and second side edges (15a, 15b) extending in said longitudinal direction, and first and second ends (16a, 16b) extending in said transverse direction;
said packing sheet being divided by first and second folds (17, 18) extending in said transverse direction from said first edge (15a) to said second edge (15b) and spaced apart from each other in said longitudinal direction into first, second and third regions (19, 20, 21);
said breast milk absorbent pad further having first and second sections (2A, 2B) defined by folding said pad into two with said skin-contactable surface (7) inside, and a temporary fastening zone (11) formed by adhesive on said skin-noncontactable surface (5) of said first section aside toward said upper end in order to enable fastening of said breast milk absorbent pad to a brassiere;
said first region (19) of said packing sheet (3) being folded back toward said skin-noncontactable surface in said first section of said breast milk absorbent pad, and fastened, in this folded state, to the temporary fastening zone (11) by a peel ply (12, 30) provided on an inner surface of said first region (19) of said packing sheet opposed to said temporary fastening zone (11), and said third region (21) of said packing sheet being folded back toward said second region (20) so that a distal end of said third region (21) extends between said first region (19) and said second region (20) of said breast milk absorbent pad (2); and
said first, second and third region (19, 20, 21) being sealed together along said first and second side edges (15a, 15b) of said packing sheet to form seal lines (26a, 26b).

2. The packed breast milk absorbent pad as claimed in claim 1, wherein said peel ply (30) is formed by a releasable sheet (12) permanently fastened to the inner surface of said first region (19) of said packing sheet (3A) by a permanent fastening zone (13) formed by adhesive.

3. The packed breast milk absorbent pad as claimed in claim 1 or claim 2, wherein said peel ply (30) is formed by a coating of an adhesive.

4. The packed breast milk absorbent pad as claimed in claim 2, wherein an end of said permanent fastening zone (13) for said releasable sheet (12) aside toward said upper end (10a) of said breast milk absorbent pad extends outward beyond an end (11a) of said temporary fastening zone (11) aside toward said upper end (10a) of said breast milk absorbent pad.

5. The packed breast milk absorbent pad as claimed in claim 2 or claim 4, wherein the peel strength of said releasable sheet (12) with respect to said temporary fastening zone (11) is in the range of 0.1 to 0.5 N and the peel strength of the seal along said first and second side edges (15a, 15b) of said packing sheet is in the range of 1.0 to 2.2 N.

6. The packed breast milk absorbent pad as claimed in any one of claims 1 to 5, wherein said first end (16a) of said packing sheet defines an unpacking outlet (24) for said breast milk absorbent pad wherein the unpacking outlet (24) is temporarily bonded to an outer surface of said third region (21) of said sheet.

7. The packed breast milk absorbent pad as claimed in any one of claims 1 to 5, wherein said first end (16a) of said packing sheet defines an unpacking outlet (24) for said breast milk absorbent pad wherein the unpacking outlet (24) is free rather than being temporarily bonded to the outer surface of said third region (21) of said sheet.

8. A method for packing a breast milk absorbent pad comprising a breast milk absorbent pad (2) having upper and lower ends (10a, 10b), a skin-contactable surface (7) as well as a skin-noncontactable surface (5) into an individual packing envelope (3) formed of a packing sheet (3A), said method comprising the steps of:
providing, as stock material for said individual packing envelope, a heat-sealable packing sheet (3A) having a longitudinal direction (X), a transverse direction (Y), first and second side edges (15a, 15b) extending in said longitudinal direction, and first and second ends (16a, 16b) extending in said transverse direction;
defining first, second and third regions (19, 20, 21) on said packing sheet by first and second folds (17, 18) extending in the transverse direction from said first edge (15a) to said second edge (15b) and spaced apart from each other in said longitudinal direction;
laying said breast milk absorbent pad upon said packing sheet so as to extend astride said first fold (17) in said longitudinal direction and thereby to lie on both said first and second regions (19, 20) of said packing sheet, then folding said third region (21) of said packing sheet along said second fold (18) toward said second region (20)and thereafter folding said first region (19) of said packing sheet together with said breast milk absorbent pad along said first fold (17) toward said second region (20) so as to fold said breast milk absorbent pad into first and second sections (2A, 2B) with said skin-contactable surface (7) inside, whereby a distal end of said third region (21) extends between said first region (19) and said second region (20) of said breast milk absorbent pad (2); - whereupon said first region (19) of said packing sheet is temporarily fixed to said skin-noncontactable surface of said breast milk absorbent pad in said first section (2A) thereof aside toward said upper end (10a) by intermediary of a temporary fastening zone (11) formed by a pressure-sensitive adhesive on said skin-noncontactable surface to enable temporary fixing of said breast milk absorbent pad to a brassiere, and by intermediary of a peel ply (12, 30) formed on said first region (19) of a said packing sheet on a surface thereof opposed to said temporary fastening zone (11); and
sealing said first and second regions (19, 20) of said packing sheet together along said first and second side edges (15a, 15b) thereof so as to form seal lines (26a, 26b).

9. The method as claimed in claim 8, wherein said seal is provided in the form of a heat seal.

## Patentansprüche

1. Eine verpackte, absorbierende Stilleinlage (1), die Folgendes beinhaltet:
eine absorbierende Stilleinlage (2) mit einem oberen und unteren Ende (10a, 10b), einer hautberührbaren Oberfläche (7) sowie einer nicht hautberührbaren Oberfläche (5);
wobei die Einlage **dadurch gekennzeichnet ist, dass**:
eine individuelle Verpackungshülle (3), die die absorbierende Stilleinlage darin verpackt, aus einer heißsiegelbaren Verpackungsschicht (3A) mit einer Längsrichtung (X), einer Querrichtung (Y), einer ersten und zweiten Seitenkante (15a, 15b), die sich in der Längsrichtung erstrecken, und einem ersten und zweiten Ende (16a, 16b), die sich in der Querrichtung erstrecken, gebildet ist;
wobei die Verpackungsschicht durch eine erste und zweite Falte (17, 18), die sich in der Querrichtung von der ersten Kante (15a) zu der zweiten Kante (15b) erstrecken und in der Längsrichtung mit Abstand voneinander angeordnet sind, in eine erste, zweite und dritte Region (19, 20, 21) unterteilt ist;
wobei die absorbierende Stilleinlage ferner einen ersten und zweiten Teilabschnitt (2A, 2B), die definiert werden, indem die Einlage mit der hautberührbaren Oberfläche (7) im Inneren zusammengefaltet wird, und ein temporäres Befestigungsfeld (11) aufweist, das durch Klebstoff auf der nicht hautberührbaren Oberfläche (5) des ersten Teilabschnitts seitlich zum oberen Ende hin gebildet ist, um das Befestigen der absorbierenden Stilleinlage an einem Büstenhalter zu ermöglichen;
wobei die erste Region (19) der Verpackungsschicht (3) zu der nicht hautberührbaren Oberfläche in dem ersten Teilabschnitt der absorbierenden Stilleinlage hin zurück gefaltet ist und in diesem gefalteten Zustand durch eine Abziehlage (12, 30), die auf einer inneren Oberfläche der ersten Region (19) der Verpackungsschicht, gegenüber dem temporären Befestigungsfeld (11), bereitgestellt ist, an dem temporären Befestigungsfeld (11) befestigt ist, und wobei die dritte Region (21) der Verpackungsschicht zu der zweiten Region (20) hin zurück gefaltet ist, so dass sich zwischen der ersten Region (19) und der zweiten Region (20) der absorbierenden Stilleinlage (2) ein distales Ende der dritten Region (21) erstreckt; und
wobei die erste, zweite und dritte Region (19, 20, 21) entlang der ersten und zweiten Seitenkante (15a, 15b) der Verpackungsschicht miteinander versiegelt sind, um Versiegelungslinien (26a, 26b) zu bilden.

2. Verpackte, absorbierende Stilleinlage gemäß Anspruch 1, wobei die Abziehlage (30) durch eine lösbare Schicht (12) gebildet ist, die durch ein permanentes Befestigungsfeld (13), das durch Klebstoff gebildet ist, permanent an der inneren Oberfläche der ersten Region (19) der Verpackungsschicht (3A) befestigt ist.

3. Verpackte, absorbierende Stilleinlage gemäß Anspruch 1 oder Anspruch 2, wobei die Abziehlage (30) durch eine Beschichtung aus einem Klebstoff gebildet ist.

4. Verpackte, absorbierende Stilleinlage gemäß Anspruch 2, wobei sich ein Ende des permanenten Befestigungsfeldes (13) für die lösbare Schicht (12) seitlich zum oberen Ende (10a) der absorbierenden Stilleinlage hin nach außen über ein Ende (11a) des temporären Befestigungsfeldes (11) seitlich zum oberen Ende (10a) der absorbierenden Stilleinlage hinaus erstreckt.

5. Verpackte, absorbierende Stilleinlage gemäß Anspruch 2 oder Anspruch 4, wobei die Abziehfestigkeit der lösbaren Schicht (12) in Bezug auf das temporäre Befestigungsfeld (11) in dem Bereich von 0,1 bis 0,5 N liegt und die Abziehfestigkeit der Versiegelung entlang der ersten und zweiten Seitenkante (15a, 15b) der Verpackungsschicht in dem Bereich von 1,0 bis 2,2 N liegt.

6. Verpackte, absorbierende Stilleinlage gemäß einem der Ansprüche 1 bis 5, wobei das erste Ende (16a) der Verpackungsschicht einen Auspackauslass (24) für die absorbierende Stilleinlage definiert, wobei der Auspackauslass (24) temporär an eine äußere Oberfläche der dritten Region (21) der Schicht gebunden ist.

7. Verpackte, absorbierende Stilleinlage gemäß einem der Ansprüche 1 bis 5, wobei das erste Ende (16a) der Verpackungsschicht einen Auspackauslass (24) für die absorbierende Stilleinlage definiert, wobei der Auspackauslass (24) ungebunden ist, statt temporär an die äußere Oberfläche der dritten Region (21) der Schicht gebunden zu sein.

8. Ein Verfahren zum Verpacken einer absorbierenden Stilleinlage, die eine absorbierende Stilleinlage (2) mit einem oberen und unteren Ende (10a, 10b), einer hautberührbaren Oberfläche (7) sowie einer nicht hautberührbaren Oberfläche (5) beinhaltet, in einer individuellen Verpackungshülle (3), die aus einer Verpackungsschicht (3A) gebildet ist, wobei das Verfahren die folgenden Schritte beinhaltet:
Bereitstellen, als Ausgangsmaterial für die individuelle Verpackungshülle, einer heißsiegelbaren Verpackungsschicht (3A) mit einer Längsrichtung (X), einer Querrichtung (Y), einer ersten und zweiten Seitenkante (15a, 15b), die sich in der Längsrichtung erstrecken, und einem ersten und zweiten Ende (16a, 16b), die sich in der Querrichtung erstrecken;
Definieren einer ersten, zweiten und dritten Region (19, 20, 21) auf der Verpackungsschicht durch eine erste und zweite Falte (17, 18), die sich in der Querrichtung von der ersten Kante (15a) zu der zweiten Kante (15b) erstrecken und in der Längsrichtung mit Abstand voneinander angeordnet sind;
Legen der absorbierenden Stilleinlage auf die Verpackungsschicht, so dass sie sich in der Längsrichtung beidseitig der ersten Falte (17) erstreckt und dadurch auf sowohl der ersten als auch der zweiten Region (19, 20) der Verpackungsschicht liegt, dann Falten der dritten Region (21) der Verpackungsschicht entlang der zweiten Falte (18) zu der zweiten Region (20) hin und danach Falten der ersten Region (19) der Verpackungsschicht zusammen mit der absorbierenden Stilleinlage entlang der ersten Falte (17) zu der zweiten Region (20) hin, um die absorbierende Stilleinlage mit der hautberührbaren Oberfläche (7) im Inneren in einen ersten und zweiten Teilabschnitt (2A, 2B) zu falten, wodurch sich ein distales Ende der dritten Region (21) zwischen der ersten Region (19) und der zweiten Region (20) der absorbierenden Stilleinlage (2) erstreckt; woraufhin die erste Region (19) der Verpackungsschicht durch das Hilfsmittel eines temporären Befestigungsfeldes (11), das durch einen Haftkleber auf der nicht hautberührbaren Oberfläche gebildet ist, um eine temporäre Fixierung der absorbierenden Stilleinlage an einem Büstenhalter zu ermöglichen, und durch das Hilfsmittel einer Abziehlage (12, 30), die auf der ersten Region (19) einer besagten Verpackungsschicht auf einer Oberfläche davon, gegenüber dem temporären Befestigungsfeld (11), gebildet ist, temporär an der nicht hautberührbaren Oberfläche der absorbierenden Stilleinlage in dem ersten Teilabschnitt (2A) davon seitlich zum oberen Ende (10a) hin fixiert wird; und
Miteinanderversiegeln der ersten und zweiten Region (19, 20) der Verpackungsschicht entlang der ersten und zweiten Seitenkante (15a, 15b) davon, um Versiegelungslinien (26a, 26b) zu bilden.

9. Verfahren gemäß Anspruch 8, wobei die Versiegelung in der Form einer Heißsiegelung bereitgestellt wird.

## Revendications

1. Un coussinet emballé absorbant le lait maternel (1) comprenant :
un coussinet absorbant le lait maternel (2) ayant des extrémités supérieure et inférieure (10a, 10b), une surface pouvant être en contact avec la peau (7) ainsi qu'une surface ne pouvant pas être en contact avec la peau (5) ; ledit coussinet étant **caractérisé en ce que** :
une enveloppe d'emballage individuelle (3) emballant ledit coussinet absorbant le lait maternel dans celle-ci est formée d'un feuillet d'emballage thermoscellable (3A) ayant une direction longitudinale (X), une direction transversale (Y), des premier et deuxième bords latéraux (15a, 15b) s'étendant dans ladite direction longitudinale, et des première et deuxième extrémités (16a, 16b) s'étendant dans ladite direction transversale ;
ledit feuillet d'emballage étant divisé par des premier et deuxième plis (17, 18) s'étendant dans ladite direction transversale dudit premier bord (15a) audit deuxième bord (15b) et espacés l'un de l'autre dans ladite direction longitudinale pour former des première, deuxième et troisième régions (19, 20, 21) ;
ledit coussinet absorbant le lait maternel ayant en outre des première et deuxième sections (2A, 2B) définies en pliant ledit coussinet en deux avec ladite surface pouvant être en contact avec la peau (7) à l'intérieur, et une zone de fixation temporaire (11) formée par adhésif sur ladite surface ne pouvant pas être en contact avec la peau (5) de ladite première section de côté vers ladite extrémité supérieure afin de permettre de fixer ledit coussinet absorbant le lait maternel à un soutien-gorge ;
ladite première région (19) dudit feuillet d'emballage (3) étant repliée vers ladite surface ne pouvant pas être en contact avec la peau dans ladite première section dudit coussinet absorbant le lait maternel, et fixée, dans cet état plié, à la zone de fixation temporaire (11) par une pellicule pelable (12, 30) prévue sur une surface interne de ladite première région (19) dudit feuillet d'emballage opposée à ladite zone de fixation temporaire (11), et ladite troisième région (21) dudit feuillet d'emballage étant repliée vers ladite deuxième région (20) de sorte qu'une extrémité distale de ladite troisième région (21) s'étende entre ladite première région (19) et ladite deuxième région (20) dudit coussinet absorbant le lait maternel (2) ; et
ladite première, ladite deuxième et ladite troisième région (19, 20, 21) étant scellées ensemble le long desdits premier et deuxième bords latéraux (15a, 15b) dudit feuillet d'emballage pour former des lignes de scellement (26a, 26b).

2. Le coussinet emballé absorbant le lait maternel tel que revendiqué dans la revendication 1, dans lequel ladite pellicule pelable (30) est formée par un feuillet détachable (12) fixé de façon permanente à la surface interne de ladite première région (19) dudit feuillet d'emballage (3A) par une zone de fixation permanente (13) formée par adhésif.

3. Le coussinet emballé absorbant le lait maternel tel que revendiqué dans la revendication 1 ou la revendication 2, dans lequel ladite pellicule pelable (30) est formée par un revêtement d'un adhésif.

4. Le coussinet emballé absorbant le lait maternel tel que revendiqué dans la revendication 2, dans lequel une extrémité de ladite zone de fixation permanente (13) destinée audit feuillet détachable (12) de côté vers ladite extrémité supérieure (10a) dudit coussinet absorbant le lait maternel s'étend vers l'extérieur au-delà d'une extrémité (11a) de ladite zone de fixation temporaire (11) de côté vers ladite extrémité supérieure (10a) dudit coussinet absorbant le lait maternel.

5. Le coussinet emballé absorbant le lait maternel tel que revendiqué dans la revendication 2 ou la revendication 4, dans lequel la résistance au pelage dudit feuillet détachable (12) par rapport à ladite zone de fixation temporaire (11) est comprise dans la gamme allant de 0,1 à 0,5 N et la résistance au pelage du scellement le long desdits premier et deuxième bords latéraux (15a, 15b) dudit feuillet d'emballage est comprise dans la gamme allant de 1,0 à 2,2 N.

6. Le coussinet emballé absorbant le lait maternel tel que revendiqué dans une quelconque des revendications 1 à 5, dans lequel ladite première extrémité (16a) dudit feuillet d'emballage définit une sortie de déballage (24) destinée au coussinet absorbant le lait maternel, la sortie de déballage (24) étant temporairement liée à une surface externe de ladite troisième région (21) dudit feuillet.

7. Le coussinet emballé absorbant le lait maternel tel que revendiqué dans une quelconque des revendications 1 à 5, dans lequel ladite première extrémité (16a) dudit feuillet d'emballage définit une sortie de déballage (24) destinée au coussinet absorbant le lait maternel, la sortie de déballage (24) étant libre plutôt que temporairement liée à la surface externe de ladite troisième région (21) dudit feuillet.

8. Une méthode destinée à emballer un coussinet absorbant le lait maternel comprenant un coussinet absorbant le lait maternel (2) ayant des extrémités supérieure et inférieure (10a, 10b), une surface pouvant être en contact avec la peau (7) ainsi qu'une surface ne pouvant pas être en contact avec la peau (5) dans une enveloppe d'emballage individuelle (3) formée d'un feuillet d'emballage (3A), ladite méthode comprenant les étapes consistant à :
fournir, comme matériau brut destiné à ladite enveloppe d'emballage individuelle, un feuillet d'emballage thermoscellable (3A) ayant une direction longitudinale (X), une direction transversale (Y), des premier et deuxième bords latéraux (15a, 15b) s'étendant dans ladite direction longitudinale, et des première et deuxième extrémités (16a, 16b) s'étendant dans ladite direction transversale ;
définir des première, deuxième et troisième régions (19, 20, 21) sur ledit feuillet d'emballage par des premier et deuxième plis (17, 18) s'étendant dans la direction transversale dudit premier bord (15a) audit deuxième bord (15b) et espacés l'un de l'autre dans ladite direction longitudinale ;
poser ledit coussinet absorbant le lait maternel sur ledit feuillet d'emballage de façon à ce qu'il soit étendu à cheval sur ledit premier pli (17) dans ladite direction longitudinale et qu'il repose de ce fait sur à la fois ladite première et ladite deuxième région (19, 20) dudit feuillet d'emballage, puis plier ladite troisième région (21) dudit feuillet d'emballage le long dudit deuxième pli (18) vers ladite deuxième région (20) et, ensuite, plier ladite première région (19) dudit feuillet d'emballage de pair avec ledit coussinet absorbant le lait maternel le long dudit premier pli (17) vers ladite deuxième région (20) de façon à plier ledit coussinet absorbant le lait maternel pour former des première et deuxième sections (2A, 2B) avec ladite surface pouvant être en contact avec la peau (7) à l'intérieur, grâce à quoi une extrémité distale de ladite troisième région (21) s'étend entre ladite première région (19) et ladite deuxième région (20) dudit coussinet absorbant le lait maternel (2) ; après quoi ladite première région (19) dudit feuillet d'emballage est temporairement assujettie sur ladite surface ne pouvant pas être en contact avec la peau dudit coussinet absorbant le lait maternel dans ladite première section (2A) de celui-ci de côté vers ladite extrémité supérieure (10a) par l'intermédiaire d'une zone de fixation temporaire (11) formée par un adhésif sensible à la pression sur ladite surface ne pouvant pas être en contact avec la peau pour permettre un assujettissement temporaire dudit coussinet absorbant le lait maternel sur un soutien-gorge, et par l'intermédiaire d'une pellicule pelable (12, 30) formée sur ladite première région (19) d'un dit feuillet d'emballage sur une surface de celle-ci opposée à ladite zone de fixation temporaire (11) ; et
sceller lesdites première et deuxième régions (19, 20) dudit feuillet d'emballage ensemble le long desdits premier et deuxième bords latéraux (15a, 15b) de celles-ci de façon à former des lignes de scellement (26a, 26b).

9. La méthode telle que revendiquée dans la revendication 8, dans laquelle ledit scellement est prévu sous la forme d'un thermoscellage.
